# EUROPEAN PATENT APPLICATION

(11) **EP 3 466 340 A1**
(43) Date of publication of application: **10.04.2019**
(21) Application number: 18185143.7
(22) Date of filing: 24.07.2018
(51) Int. Cl.: A61B 6/06, A61B 6/00

(54) **X-RAY IMAGING DEVICE**

(30) Priority: 03.10.2017 JP 2017193575
(71) Applicant: Shimadzu Corporation, Kyoto-shi Kyoto 604-8511 (JP)
(72) Inventor: Sakamoto, Yuki, Kyoto-shi, Kyoto 604-8511 (JP); Okamoto, Takeshi, Kyoto-shi, Kyoto 604-8511 (JP)
(74) Representative: Müller-Boré & Partner Patentanwälte PartG mbB

(57) **Abstract**

[Problem] To provide an X-ray imaging device that eliminates not knowing whether stored imaging conditions are for a previous test subject or for a current test subject.

[Resolution Means] A reset instruction unit 57, in between a sequence of imaging a previous or next test subject M and a sequence of imaging a current test subject M, erases imaging conditions stored in two condition reproduction memories 53, 54 in conjunction with a preset operation by an operation unit 42. As a result, when the test subject M changes, the imaging conditions stored in the two condition reproduction memories 53, 54 are deleted without fail. Thus, it is possible to determine for certain that the imaging conditions stored in the two condition reproduction memories 53, 54 are conditions that are suitable for the current test subject M. Therefore, it is possible to eliminate not knowing whether the stored imaging conditions are for a previous test subject M or for the current test subject M.

## Description

### [Technical Field]

The present invention relates to an X-ray imaging device that images a test subject by irradiating the test subject with X rays.

### [Background Art]

For example, when performing a procedure during surgery, an X-ray imaging device for surgery (hereinafter, called an "X-ray imaging device," as appropriate) is used. The X-ray imaging device includes: an X-ray tube to which a collimator is attached; an X-ray detector; a C-arm that supports the X-ray tube and the X-ray detector; and a monitor, with the C-arm being provided on a dolly. During the procedure, an operator freely operates the C-arm and obtains an X-ray image of a desired portion of the test subject.

An operation panel of the X-ray imaging device, as in Patent Literature 1, for example, includes: a memory key; and condition reproduction memory (a memory key storage unit) that is matched up with this memory key. When the memory key is held down, the imaging conditions of the X-ray imaging device when the memory key is being held down are stored in the condition reproduction memory. At such time, the stored imaging conditions are set as imaging conditions A. Examples of imaging conditions, include, for example: the angle of rotation of a camera; the X-ray conditions; the aperture (open state) of the collimator; and the like. Thereafter, when the operator wants to change the imaging conditions and once again return to the imaging conditions A stored in the condition reproduction memory, the operator presses the memory key normally. By so doing, the X-ray imaging device is set to the imaging conditions A. It is possible to eliminate the hassle of resetting each condition (each item) when changing the imaging conditions and then returning, and to reproduce the imaging conditions in necessary situations.

### [Prior Art Literature]

### [Patent Literature]

[Patent Literature 1]
Japanese Unexamined Patent Application Publication No. 2013-017759

### [Summary of the Invention]

### [Problems to be Solved by the Invention]

However, conventional X-ray imaging devices have the following such problems. The imaging conditions stored in the condition reproduction memory are updated by operating the memory key. Thus, imaging conditions that have been stored once will continue to be stored unless updated. For example, when a procedure on a certain test subject (patient) has been completed and a procedure will be conducted on the next test subject, the operator may be unable to tell whether the conditions stored in the condition reproduction memory were set when the previous test subject was examined or were set during testing of the current test subject. This becomes more likely to occur as work progresses. When the imaging conditions for the previous test subject are mistakenly reproduced, imaging conditions that are unsuitable for the current test subject will be reproduced. In such a case, each condition must be reset one-by-one from those imaging conditions to the imaging conditions for the current test subject; thus, this will take a lot of time and effort. As a result, the operator will feel that this is a hassle.

In light of the above, an object of the present invention is to provide an X-ray imaging device that eliminates not knowing whether the stored imaging conditions are for a previous test subject or for the current test subject.

### [Means for Solving the Problems]

In order to achieve such an aim, the present invention has the following configuration. In other words, an X-ray imaging device according to the present invention includes: an X-ray source that radiates X rays toward a test subject; a collimator that is attached to the X-ray source and that adjusts an irradiation field of the X-rays radiated from the X-ray source; an X-ray detector that detects the X-rays that have passed through the test subject; a reference memory that stores imaging conditions including X-ray conditions and collimator conditions, and that is referenced during X-ray imaging; a condition reproduction memory that stores a copy of the imaging conditions stored in the reference memory; a memory switch that causes the imaging conditions stored in the reference memory to be stored in the condition reproduction memory, and that switches the imaging conditions stored in the condition reproduction memory with the imaging conditions in the reference memory; an operation unit that performs at least setting of the imaging conditions in the reference memory; and a reset instruction unit that, in between a sequence of imaging the previous or next test subject and a sequence of imaging the current test subject, deletes the imaging conditions stored in the condition reproduction memory in conjunction with a preset operation by the operation unit.

According to an X-ray imaging device of the present invention, in between a sequence of imaging the previous or next test subject and a sequence of imaging the current test subject, the reset instruction unit deletes the imaging conditions stored in the condition reproduction memory in conjunction with a preset operation of the operation unit. As a result, when the test subject changes, the imaging conditions stored in the condition reproduction memory will be deleted without fail. Thus, it is possible to determine for certain that the imaging conditions stored in the condition reproduction memory are conditions that are suitable for the current test subject. Therefore, it is possible to eliminate not knowing whether the stored imaging conditions are for a previous test subject or for the current test subject.

In addition, it is preferable in the above-mentioned X-ray imaging device that the operation unit includes: an imaging initiation switch that, when initiating the sequence of imaging the current test subject, associates an X-ray image acquired via the X-ray detector and the current test subject; and an imaging termination switch that deletes the association when terminating the sequence of imaging of the current test subject, with the reset instruction unit deleting the imaging conditions stored in the condition reproduction memory in conjunction with the operation of canceling the association performed by the imaging termination switch of the operation unit.

The imaging termination switch cancels the association, which was performed using the imaging initiation switch, between the X-ray image acquired via the X-ray detector and the current test subject. The reset instruction unit deletes the imaging conditions stored in the condition reproduction memory in conjunction with the operation of canceling the association performed by the imaging termination switch. As a result, when changing from the current test subject to the next test subject, the imaging conditions stored in the condition reproduction memory are deleted without fail. Thus, it is possible to determine for certain that the imaging conditions stored in the condition reproduction memory are conditions that are suitable for the current test subject.

In addition, it is preferable in the above-mentioned X-ray imaging device that the operation unit includes: the imaging initiation switch that, when initiating the sequence of imaging the current test subject, associates the X-ray image acquired via the X-ray detector and the current test subject; and the imaging termination switch that cancels the association when terminating the sequence of imaging the current test subject, wherein the reset instruction unit deletes the imaging conditions stored in the condition reproduction memory in conjunction with the operation of performing association via the imaging initiation switch of the operation unit.

The imaging initiation switch performs association between the X-ray image acquired using the X-ray detector and the current test subject. The reset instruction unit deletes the imaging conditions stored in the condition reproduction memory in conjunction with the operation that performs association via the imaging initiation switch. As a result, when changing from a previous test subject to the current test subject, the imaging conditions stored in the condition reproduction memory are deleted without fail. Thus, it is possible to determine for certain that the imaging conditions stored in the condition reproduction memory are conditions that are suitable for the current test subject.

In addition, it is preferable in the above-mentioned X-ray imaging device that the memory switch includes a status display unit, and that when the imaging conditions stored in the condition reproduction memory are deleted, the status display unit performs a display that is different from a display when the imaging conditions are stored in the condition reproduction memory. As a result, the operator is able to know that the imaging conditions stored in the condition reproduction memory have been deleted via the status display unit of the memory switch.

In addition, it is preferable that the above-mentioned X-ray imaging device further includes a notification unit that performs notification about the deletion when the imaging conditions stored in the condition reproduction memory are deleted. As a result, the operator is able to know that the imaging conditions in the condition reproduction memory have been deleted via the notification unit.

In addition, it is preferable that the above-mentioned X-ray imaging device further includes: second condition reproduction memory that stores a copy of the imaging conditions stored in the reference memory; and a second memory switch that causes the imaging conditions stored in the reference memory to be stored in the second condition reproduction memory, and that switches the imaging conditions stored in the second condition reproduction memory with the imaging conditions in the reference memory, with the reset instruction unit deleting the imaging conditions respectively stored in the condition reproduction memory and the second condition reproduction memory.

In a case in which the present invention further includes the second condition reproduction memory and the second memory switch corresponding to this, the reset instruction unit, in between a sequence of imaging the previous or next test subject and the sequence of imaging the current test subject, deletes the imaging conditions respectively stored in the condition reproduction memory and the second condition reproduction memory in conjunction with a preset operation by the operation unit. As a result, when the test subject changes, the imaging conditions respectively stored in the condition reproduction memory and the second condition reproduction memory are deleted without fail.

### [Effects of the Invention]

According to an X-ray imaging device of the present invention, it is possible to eliminate not knowing whether the stored imaging conditions are for a previous test subject or for the current test subject.

### [Brief Description of the Drawings]

[FIG. 1]

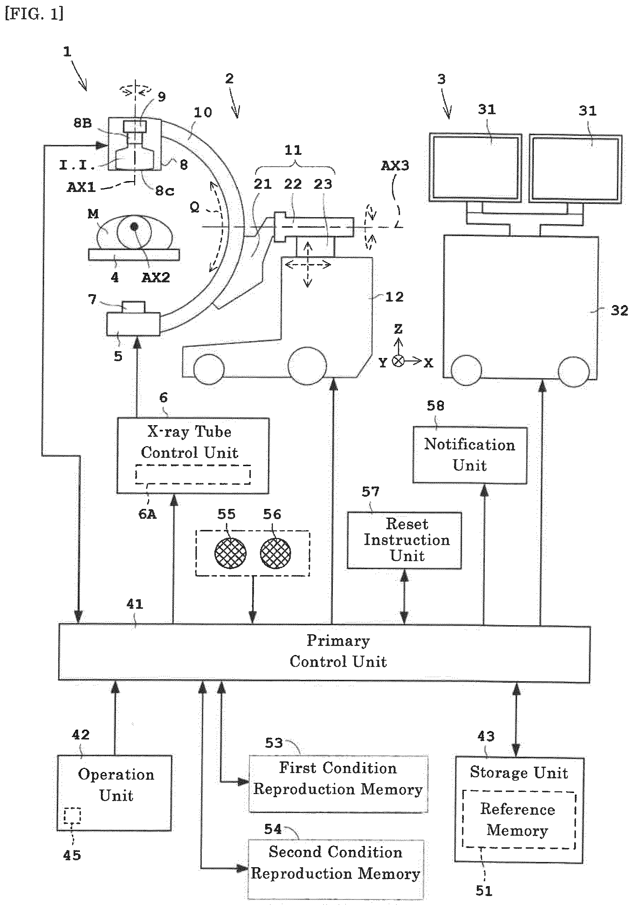

   FIG. 1 is a schematic configuration diagram of an X-ray imaging device according to Example 1.
[FIG. 2]

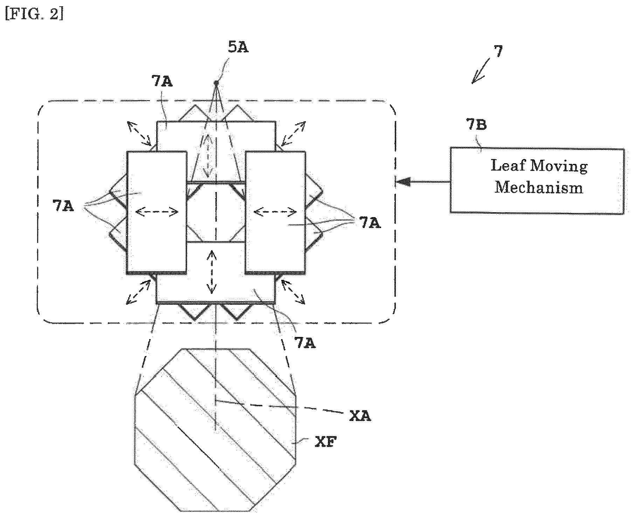

   FIG. 2 is a schematic configuration diagram of a collimator.
[FIG. 3]

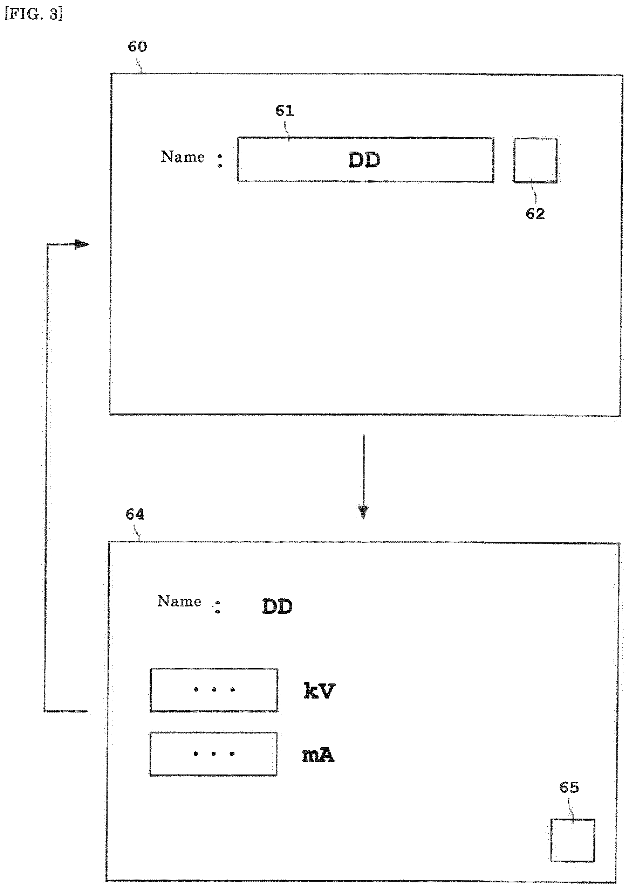

   FIG. 3 is a figure illustrating an example of an operation screen displayed on a monitor.
[FIG. 4]

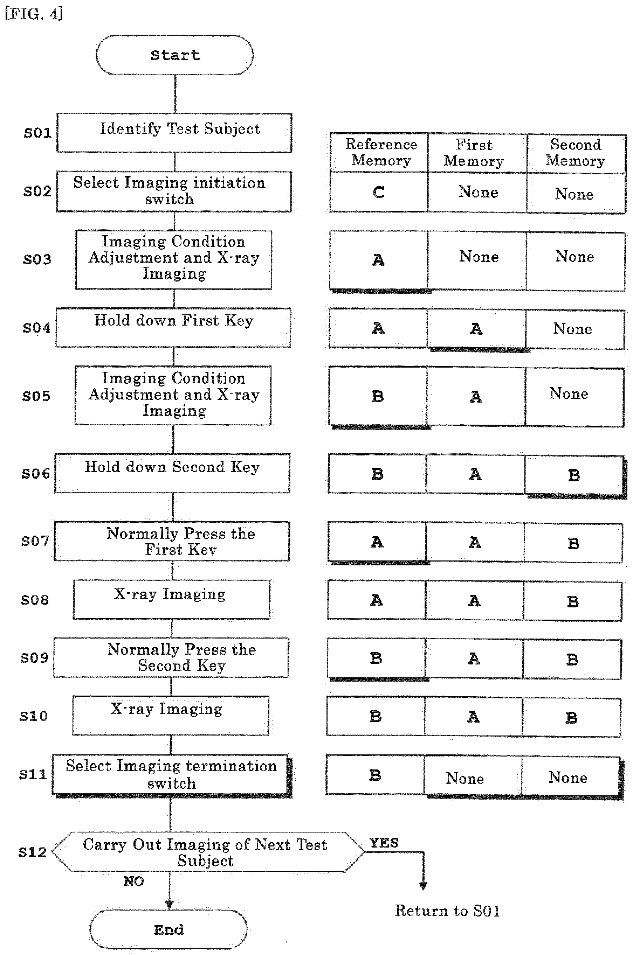

   FIG. 4 is a flow chart illustrating an operation example of the X-ray imaging device.
[FIG. 5]

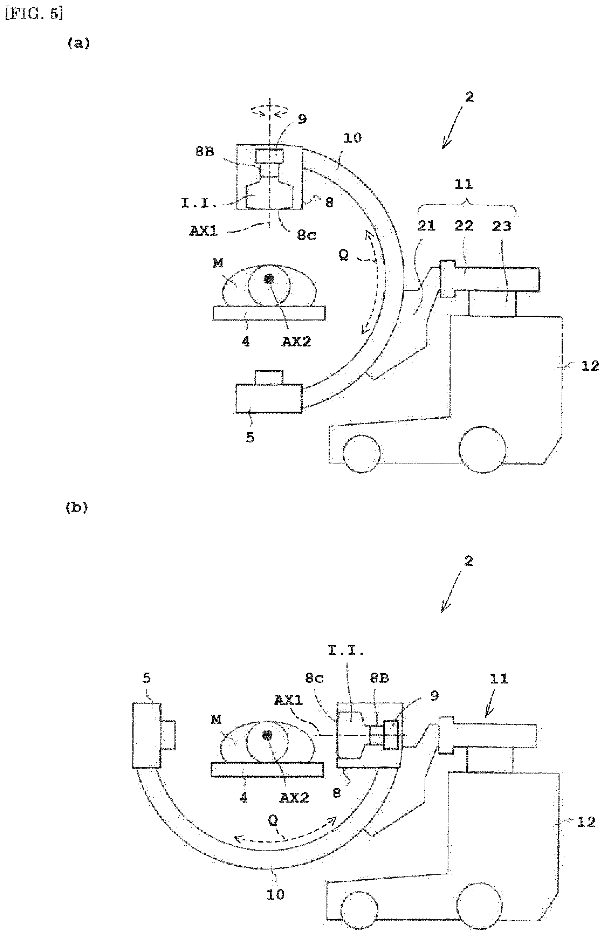

   FIGs. 5(a) and 5(b) are figures for describing the operation of the X-ray imaging device.
[FIG. 6]

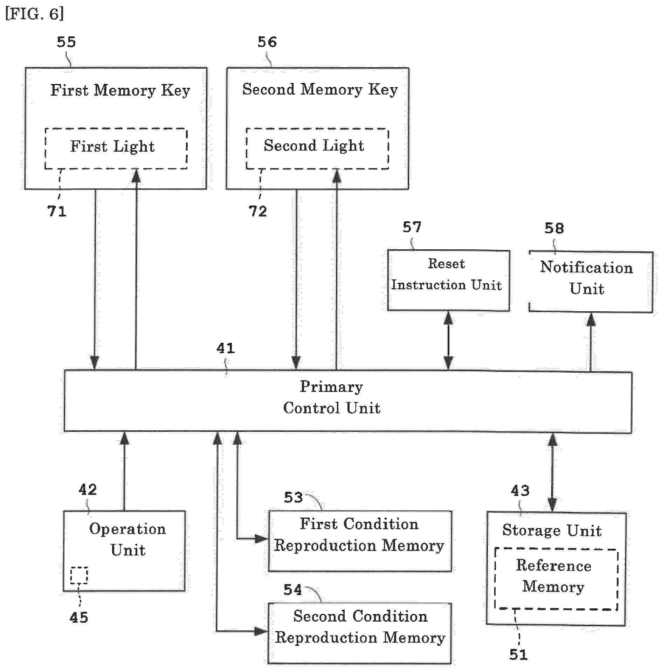

   FIG. 6 is a figure illustrating a control system for lights and the like of an X-ray imaging device according to Example 2.
[FIG. 7]

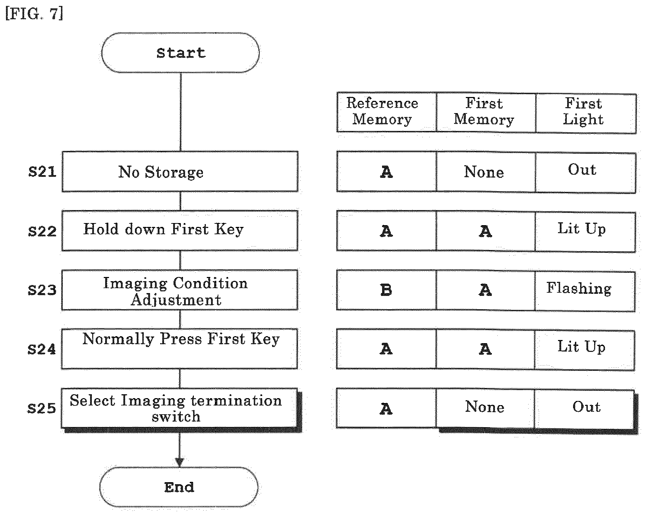

   FIG. 7 is a flow chart for describing an operation example of the lights and the like of the X-ray imaging device according to Example 2.
[FIG. 8]

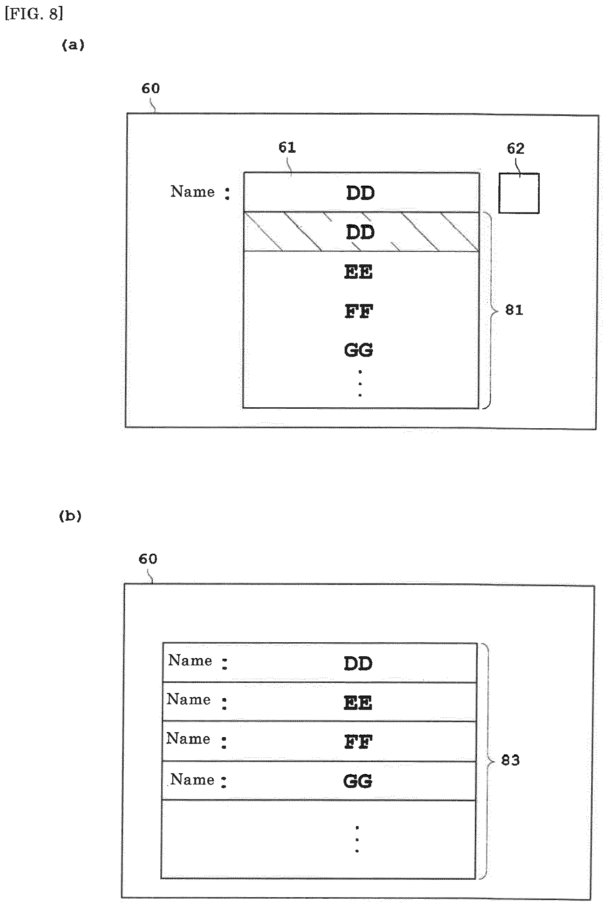

   FIGs. 8(a) and 8(b) are figures illustrating variations of the operation screen.

### [Example 1]

Example 1 of the present invention will be described below with reference to the drawings. FIG. 1 is a schematic configuration diagram of an X-ray imaging device according to Example 1. FIG. 2 is a schematic configuration diagram of a collimator.

### <Configuration of X-ray Imaging Device 1>

This description will reference FIG. 1. An X-ray imaging device 1 for surgery is of a moving type, and includes: an X-ray imaging device main body 2; a display unit 3; and a top slab 4. In addition, the X-ray imaging device main body 2 includes: an X-ray tube 5; an X-ray tube control unit 6; a collimator 7; an X-ray detector 8; and a rotation unit 9. Note that the X-ray tube 5 corresponds to the X-ray source of the present invention.

The X-ray tube 5 radiates X-rays toward a test subject M placed on the top slab 4. The X-ray tube 5 is controlled via the X-ray tube control unit 6. The X-ray tube control unit 6 includes an X-ray control circuit (not shown) and a high voltage generation unit 6A, and supplies preset tube voltage and tube current to the X-ray tube 5. In addition, the collimator 7 is attached to the X-ray tube 5.

The collimator 7 adjusts an irradiation field XF of the X-rays radiated from the X-ray tube 5. The collimator 7, as in FIG. 2, includes eight leaves 7A, for example, and forms the octagonal irradiation field XF. In other words, when two leaves 7A that face each other are set as one group, the collimator 7 includes four groups of the leaves 7A. Each leaf 7A is moved in a straight line via a leaf moving mechanism 7B. The leaf moving mechanism 7B includes an electric motor. Note that the leaves 7A of the collimator 7 illustrated in FIG. 2 are constituted of eight leaves; however, they may be constituted of another number of leaves, such as two leaves, four leaves, six leaves, or the like. In addition, the movement of each leaf 7A may not be a straight-line movement and may be a curved line movement. In addition, in FIG. 2, the reference numeral 5A represents an X-ray focus formed in the X-ray tube 5, and the reference numeral XA represents an X-ray axis (an X-ray central axis).

The x-ray detector 8, as in FIG. 1, is disposed so as to face the X-ray tube 5, and detects X-rays that have passed through the test subject M. The X-ray detector 8 includes: an image intensifier I.I.; and a camera 8B (a CCD camera or CMOS camera, for example). When a straight line AX1 that is orthogonal to a detection surface 8C of the X-ray detector 8 is set as the axis, the rotation unit 9 rotates the camera 8B or the X-ray detector 8 about the axis AX1. In other words, the rotation unit 9 rotates about the axis AX1 that is orthogonal to the detection surface 8C of the X-ray detector 8, and changes the angle of rotation (imaging angle of rotation) for when an X-ray image is captured using the X-ray detector 8. The rotation unit 9 includes an electric motor. In addition, the X-ray detector 8 may be an FPD (flat panel detector). In such a case, the rotation unit 9 rotates the FPD about the axis AX1. Note that the image captured by the X-ray detector 8 undergoes necessary image processing, such as various types of correction, via an image processing unit (not shown).

The X-ray imaging device main body 2 further includes: a C-arm 10; an arm rotation and movement mechanism 11; and a dolly 12. The C-arm 10 is a support arm that has a C-shape. The C-arm 10 supports the X-ray tube 5, the X-ray detector 8, and the like. The C-arm 10 is configured so as to move and rotate via the arm rotation and movement mechanism 11. The arm rotation and movement mechanism 11 includes: a slide mechanism 21; a rotation mechanism 22; and a vertical/horizontal movement mechanism 23. The arm rotation and movement mechanism 11 is provided on the dolly 12.

The slide mechanism 21, as indicated by the arrow Q, slidably supports the C-arm 10 along the C shape of the C-arm 10. As a result, the X-ray tube 5 and the X-ray detector 8 rotate about the axis AX2. The rotation mechanism 22 rotatably supports the slide mechanism 21 about an axis AX3. As a result, the X-ray tube 5 and the X-ray detector 8 rotate about the axis AX3. The vertical/horizontal movement mechanism 23 moves the rotation mechanism 22 in the vertical direction (Z direction) and a horizontal direction (X direction). As a result, the X-ray tube 5 and the X-ray detector 8 move in the vertical direction (Z direction) and a horizontal direction (X direction). The slide mechanism 21 and the rotation mechanism 22 include guide members (called "guide rails" or "slide rails"). The slide mechanism 21 and the rotation mechanism 22 may include an electric motor. The vertical/horizontal movement mechanism 23 includes an electric motor.

The display unit 3 includes two monitors 31, and a dolly 32 that supports the two monitors 31. Each monitor 31 is configured of a liquid crystal display (LCD), an organic EL display, or the like, for example. The display unit 3 is configured so as to be able to perform wired communication or wireless communication with the X-ray imaging device main body 2.

The X-ray imaging device 1 further includes: a primary control unit 41; an operation unit 42; and a storage unit 43. The primary control unit 41 includes a central processing unit (CPU). The primary control unit 41 controls each configuration of the X-ray tube control unit 6, the collimator 7, the X-ray detector 8, the rotation unit 9, the arm rotation and movement mechanism 11, two memory keys 55, 56, which will be mentioned later, and the like. The operation unit 42 includes at least one of: a keyboard, a mouse, and a touch panel, for example. The touch
panel is provided on the front surface of the screen of the monitor 31, and is configured of an input device of a detection type, such as a resistive film-type, for example. The operation unit 42 performs operations, such as setting the imaging conditions and test subject information, in reference memory 51, which will be explained later, via the operator. In addition, the operation unit 42 includes an exposure switch 45. In addition, the operation unit 42 of the present working example includes the monitor 31, which displays an imaging initiation switch 62 and an imaging termination switch 65, which will be mentioned later. The storage unit 43 includes at least one of ROM (read-only memory), RAM (random-access memory), and a hard disk, for example. The storage unit 43 stores programs necessary for operating the X-ray imaging device 1, and the like.

### <Memory Function and Reset Function of Imaging Conditions>

The imaging condition memory function and reset function installed in the X-ray imaging device 1 will be described. The memory function is a function that, by storing adjusted imaging conditions, can reproduce imaging conditions even if the imaging conditions are changed. As a result, it is possible to eliminate the hassles of resetting each condition of the imaging conditions. However, there are instances in which there may still be a hassle, regardless of the fact that the memory function is a function that is able to eliminate hassle. These are instances in which it is unclear whether stored imaging conditions are for a previous test subject or the current test subject. When imaging conditions for a previous test subject are mistakenly reproduced, imaging conditions that are unsuitable for the current test subject will be reproduced. In such a case, each condition must be reset one-by-one from those imaging conditions to the imaging conditions for the current test subject; thus, this will take a lot of time. Therefore, the present invention eliminates not knowing whether the stored imaging conditions are for a previous test subject or the current test subject.

The storage unit 43 includes the reference memory 51, which stores imaging conditions. The reference memory 51 is referred to during X-ray imaging and the like. Thus, the primary control unit 41 refers to the imaging conditions stored in the reference memory 51 and then performs X-ray imaging or the like. The imaging conditions include, for example: X-ray conditions; collimator conditions; and the angle of rotation of the X-ray detector 8. The X-ray conditions include the tube voltage and the tube current. The collimator conditions include the aperture (open state) of the collimator 7. The angle of rotation of the X-ray detector 8 is the angle of rotation of the camera 8B or the X-ray detector 8 about the axis AX1. This angle of rotation is an angle of rotation with respect to a standard angle of rotation. Note that when the X-ray detector 8 is an FPD, this is the angle of rotation of the FPD about the axis AX1. The imaging conditions may include conditions other than these conditions. Examples include image processing conditions for the angle of rotation of an image, and the like, for example. In addition, when a rotation mechanism (not shown) that causes the octagonal irradiation field XF to rotate is included, this may be the angle of rotation of the collimator 7 (leaves 7A) about the X-ray axis XA. This rotation mechanism includes an electric motor.

The X-ray imaging device 1 further includes: the two condition reproduction memories 53, 54; the two memory keys 55, 56; the reset instruction unit 57; and the notification unit 58. The memory key 55 corresponds to the memory switch of the present invention, and the memory key 56 corresponds to the second memory switch.

The two condition reproduction memories 53, 54 are respectively configured of RAM or the like. The two condition reproduction memories 53, 54 respectively store a copy of the imaging conditions stored in the reference memory 51. The two memory keys 55, 56 respectively include a mechanical switch (physical switch) called a "tactile switch" or a "push switch," for example. Note that the imaging conditions stored in the two condition reproduction memories 53, 54 are information that is not associated with the test subject M identified using the screen 60, which will be mentioned later. In other words, even if the test subject M changes, the imaging conditions stored in the two condition reproduction memories 53, 54 are information that will not be updated as a result of the changes thereof.

The first memory key 55 is configured so as to, when "held down," for example, cause the imaging conditions stored in the reference memory 51 to be stored in the first condition reproduction memory 53. In addition, the first memory key 55 is configured so as to, when "pressed normally," where the pressing is shorter than when being held down, for example, cause the imaging conditions stored in the first condition reproduction memory 53 to be switched with the imaging conditions in the reference memory 51.

Similarly, the second memory key 56 is configured so as to, when "held down," for example, cause the imaging conditions stored in the reference memory 51 to be stored in the second condition reproduction memory 54. In addition, the second memory key 56 is configured so as to, when "pressed normally," where the pressing is shorter than when being held down, for example, cause the imaging conditions stored in the second condition reproduction memory 54 to be switched with the imaging conditions in the reference memory 51.

In between a sequence of imaging the previous or next test subject M and a sequence of imaging the current test subject M, the reset instruction unit 57 deletes the imaging conditions respectively stored in the two condition reproduction memories 53, 54 in conjunction with a preset operation of the operation unit 42. The reset instruction unit 57 may include a CPU, may be configured of programs and be operated by the primary control unit 41, and may be included in the primary control unit 41. Note that "a preset operation of the operation unit 42" is an operation other than the operation that deletes the imaging conditions respectively stored in the two condition reproduction memories 53, 54.

Here, the reset instruction unit 57 will be described. FIG. 3 is a figure illustrating an example of the operation screens 60, 64 displayed on the monitor 31. The screen 60 is a test subject identification screen that identifies the test subject M via test subject information such as a test subject name (a patient name, for example), a test subject ID (a patient ID, for example), and the like. The test subject identification screen 60 includes a test subject information input field 61 and the imaging initiation switch 62.

A test subject name, for example, is input into the test subject information input field 61 as test subject information. The input is performed via the operation unit 42 (a keyboard, for example) while looking at the screen 60 on the monitor 31. The imaging initiation switch 62 is a switch for performing an X-ray imaging sequence of the current test subject that corresponds to the test subject name input in the test subject information input field 61. In other words, the imaging initiation switch 62, when initiating an imaging sequence of the current test subject, performs association between the current test subject M and the X-ray image acquired by the X-ray detector 8. Note that X-ray images include moving images and still images. When the imaging initiation switch 62 is selected, the device switches to an X-ray imaging preparation screen 64.

The X-ray imaging preparation screen 64 is a screen that displays X-ray conditions such as tube voltage and tube current. Note that the X-ray conditions and the like may be edited using this screen 64, and the X-ray conditions and the like may be edited on a different screen (not shown) that has been transferred to from the screen 64. When the screen 64 is displayed on the monitor 31, X-ray imaging is executed when the exposure switch 45 is pressed. In addition, the X-ray imaging preparation screen 64 includes the imaging termination switch 65.

The imaging termination switch 65 is a switch for returning to the test subject identification screen 60. In other words, the imaging termination switch 65, when terminating an imaging sequence of the current test subject M, cancels the association between the current test subject M and the X-ray image acquired by the X-ray detector 8. When the imaging termination switch 65 is selected, the device switches from the X-ray imaging preparation screen 64 to the test subject identification screen 60. The test subject identification screen 60, the imaging initiation switch 62, the X-ray imaging preparation screen 64, and the imaging termination switch 65 are configured of programs executed by the primary control unit 41.

In the present example, the reset instruction unit 57 is configured so as to delete the imaging conditions stored in the two condition reproduction memories 53, 54 in conjunction with the operation (a preset operation) of canceling the association performed by the imaging termination switch 65 of the operation unit 42.

In addition, when the imaging conditions stored in the two condition reproduction memories 53, 54 are deleted, the notification unit 58 performs notification about the deletion. The notification unit 58 may be configured of a buzzer and speakers and perform notification about the deletion using sound or a voice. In addition, the notification unit 58 may be configured of the monitor 31 and perform notification about the deletion by performing display on the monitor 31 via text and diagrams.

### <Operation of X-ray Imaging Device 1>

FIG. 4 is a flow chart illustrating an operation example of the X-ray imaging device. The operation example of the X-ray imaging device 1 will be described while referencing FIG. 4. Note that, in FIG. 4, the first condition reproduction memory 53 is represented by "the first memory" and the second condition reproduction memory 54 is represented by "the second memory."

### [Step S01]

The test subject (patient) M is placed on the top slab 4. The operator looks at the test subject identification screen 60 that is illustrated in FIG. 3 and that is displayed on one of the two monitors 31, for example, and inputs test subject information (the test subject name, for example) into the test subject information input field 61 on the test subject identification screen 60 using the operation unit 42.

### [Step S02]

After the test subject information is input into the test subject information input field 61, the imaging initiation switch 62 is selected. When the imaging initiation switch 62 is selected, the X-ray imaging preparation screen 64 is displayed in place of the test subject identification screen 60. In other words, when the imaging initiation switch 62 is selected, the device is in a state of being in the middle of a sequence of imaging (in the middle of an inspection or in the middle of a procedure) this test subject M. In addition, the imaging initiation switch 62 performs association between the X-ray image acquired by the X-ray detector 8 and the (current) test subject input into the test subject information input field 61. Note that X-rays will not be radiated from the X-ray tube 5 merely by the imaging initiation switch 62 being selected.

Note that, as in FIG. 4, during Step S02, the reference memory 51, the first condition reproduction memory (first memory) 53, and the second condition reproduction memory (second memory) 54 are assumed to be in the following state, for example. Imaging conditions C other than the imaging conditions A, B are stored in the reference memory 51. In addition, imaging conditions have not been stored in the first condition reproduction memory 53 and the second condition reproduction memory 54.

### [Step S03]

After the imaging initiation switch 62 is selected, the imaging conditions are adjusted, and X-ray imaging is performed. This will be explained in detail. The operator, for example, changes the attitude of the C-arm 10 via the arm rotation and movement mechanism 11 such that the X-ray tube 5 and the X-ray detector 8, which face each other, are vertically aligned (see FIG. 5(a)). In addition, the operator adjusts (sets) the imaging conditions, such as the X-ray conditions, which include the tube voltage and the tube current, the aperture of the collimator 7, and the angle of rotation of the camera 8B of the X-ray detector 8, via the operation unit 42. In other words, the operator adjusts the imaging conditions stored in the reference memory 51. Note that the aperture of the collimator 7 and the angle of rotation of the camera 8B may be adjusted while X-ray fluoroscopy is being executed. During Step S03, the adjusted imaging conditions are set as the imaging conditions A. The imaging conditions A are stored in the reference memory 51. Then, when the exposure switch 45 is pressed, the primary control unit 41 refers to the reference memory 51, and executes X-ray imaging based on the imaging conditions A stored in the reference memory 51.

When the exposure switch 45 is pressed, the X-ray tube 5 radiates X-rays toward the test subject M based on the X-ray conditions. The collimator 7 illustrated in FIG. 2 adjusts the X-rays radiated from the X-ray tube 5 to the preset aperture and octagonal irradiation field XF. The x-ray detector 8 that is disposed so as to face the X-ray tube 5 detects X-rays that have passed through the test subject M. The image intensifier I.I. detects the X-rays and changes them into an image, and the camera 8B captures the image changed into an image by the image intensifier I.I. The X-ray image (moving image or still image) captured by the camera 8B undergoes prescribed image processing. The X-ray image that undergoes the prescribed image processing is displayed on the monitor 31 or is set as the X-ray image of the test subject identified on the test subject identification screen 60 and is stored in the storage unit 43.

### [Step S04]

After the imaging conditions are adjusted and X-ray imaging is performed during Step S03, the first memory key 55 is operated (held down) and the imaging conditions A stored in the reference memory 51 are stored in the first condition reproduction memory (first memory) 53. In other words, the imaging conditions A, such as the X-ray conditions, which include the tube voltage and the tube current, the aperture of the collimator 7, and the angle of rotation of the camera 8B of the X-ray detector 8, are stored in the first condition reproduction memory 53.

### [Step S05]

After recording the imaging conditions A in the first condition reproduction memory 53, the operator, for example, changes the attitude of the C-arm 10 via the arm rotation and movement mechanism 11 such that the X-ray tube 5 and the X-ray detector 8, which face each other, are horizontally aligned, as in FIG. 5(b). The operator adjusts the imaging conditions stored in the reference memory 51 via the operation unit 42 so as to correspond to the attitude of the C-arm 10. During Step S05, the adjusted imaging conditions are set as the imaging conditions B. The imaging conditions B are stored in the reference memory 51. When the exposure switch 45 is pressed, the primary control unit 41 refers to the reference memory 51, and executes X-ray imaging based on the imaging conditions B stored in the reference memory 51. The acquired X-ray image is displayed on the monitor 31 or is set as being an X-ray image of the test subject M identified on the test subject identification screen 60 and is stored in the storage unit 43.

### [Step S06]

After the imaging conditions are adjusted and X-ray imaging is performed during Step S05, the second memory key 56 is operated (held down) and the imaging conditions B stored in the reference memory 51 are stored in the second condition reproduction memory (second memory) 54. In other words, the imaging conditions B, such as the X-ray conditions, which include the tube voltage and the tube current, the aperture of the collimator 7, and the angle of rotation of the camera 8B of the X-ray detector 8, are stored in the second condition reproduction memory 54.

### [Step S07]

After storing the imaging conditions B in the second condition reproduction memory 54, the operator once again changes the attitude of the C-arm 10 to vertical, as in FIG. 5(a). The operator operates (presses normally) the first memory key 55, and switches the imaging conditions A stored in the first condition reproduction memory 53 and the imaging conditions B, for example, in the reference memory 51. Thus, the imaging conditions A read from the first condition reproduction memory 53 are stored in the reference memory 51 in place of the imaging conditions B. For example, the aperture of the collimator, the angle of rotation of the camera 8B, and the X-ray condition information, such as the tube voltage and the tube current displayed on the screen 64, automatically change based on the imaging conditions A. Note that the imaging conditions A stored in the reference memory 51 may be appropriately fine-tuned.

### [Step S08]

When the exposure switch 45 is pressed, the primary control unit 41 executes X-ray imaging based on the imaging conditions A stored in the reference memory 51. The acquired X-ray image is displayed on the monitor 31 or is set as being an X-ray image of the test subject M identified on the test subject identification screen 60 and is stored in the storage unit 43.

### [Step S09]

After the X-ray imaging of Step S08, the operator once again changes the attitude of the C-arm 10 to horizontal, as in FIG. 5(b). The operator operates (presses normally) the second memory key 56, and switches the imaging conditions B stored in the second condition reproduction memory 53 and the imaging conditions A in the reference memory 51. Thus, the imaging conditions B read from the second condition reproduction memory 54 are stored in the reference memory 51 in place of the imaging conditions A. For example, the aperture of the collimator, the angle of rotation of the camera 8B, and the X-ray condition information, such as the tube voltage and the tube current displayed on the screen 64, automatically change based on the imaging conditions B. Note that the imaging conditions B stored in the reference memory 51 may be appropriately fine-tuned.

### [Step S10]

When the exposure switch 45 is pressed, the primary control unit 41 executes X-ray imaging based on the imaging conditions B stored in the reference memory 51. The acquired X-ray image is displayed on the monitor 31 or is set as being an X-ray image of the test subject M identified on the test subject identification screen 60 and is stored in the storage unit 43.

### [Step S11]

After the X-ray imaging of Step S10, the operator, in order to end the X-ray imaging sequences of Steps S03, S05, S08, and S10, selects the imaging termination switch 65 of the X-ray imaging preparation screen 64 illustrated in FIG. 3. When the imaging termination switch 65 is selected, the screen switches from the X-ray imaging preparation screen 64 to the test subject identification screen 60. In conjunction with this, the association between the X-ray image acquired using the X-ray detector 8 and the current test subject M is canceled. In other words, the device cancels the state of being in the middle of a sequence of imaging (in the middle of an inspection or in the middle of a procedure) this test subject.

In addition, when the imaging termination switch 65 is selected, the reset instruction unit 57 acts in conjunction with this. In other words, the reset instruction unit 57 deletes the imaging conditions A,B stored in the two condition reproduction memories 53, 54 in conjunction with the operation of canceling the association between the current test subject M and the X-ray image acquired by the X-ray detector 8, which is performed by the imaging termination switch 65 of the operation unit 42. As a result, as in FIG. 4, the device changes to a state in which imaging conditions are not stored in the two condition reproduction memories 53, 54.

Note that, when the imaging conditions in the two condition reproduction memories 53, 54 are deleted and no imaging conditions are being stored, the primary control unit 41 performs control so as to, even when the respective two memory keys 55, 56 are operated (pressed normally), not execute the operation of switching the imaging conditions in the reference memory 51. Thus, it is configured such that nothing happens even when the two memory keys 55, 56 are respectively operated (pressed normally).

### [Step S12]

After the imaging conditions A, B stored in the two respective condition reproduction memories 53, 54 are deleted, the device returns to Step S01 when performing imaging of the next test subject M.

According to the present example, the imaging termination switch 65 cancels the association between the X-ray image acquired via the X-ray detector 8 and the current test subject M, which was performed using the imaging initiation switch 62. In between a sequence of imaging the next test subject M and a sequence of imaging the current test subject M, the reset instruction unit 57 deletes the imaging conditions A, B stored in the two condition reproduction memories 53, 54 in conjunction with the operation of canceling association, which is performed by the imaging termination switch 65. As a result, when changing between the current test subject M and the next test subject M, the imaging conditions A, B stored in the two condition reproduction memories 53, 54 are deleted without fail. Thus, it is possible to determine for certain that the imaging conditions A, B stored in the two condition reproduction memories 53, 54 are conditions that are suitable for the current test subject. Therefore, it is possible to eliminate not knowing whether the stored imaging conditions are for a previous test subject M or for the current test subject M.

Thus, the imaging conditions stored in the two condition reproduction memories 53, 54 during the imaging sequence of the previous test subject M are not mistakenly reproduced during the imaging sequence of the current test subject M and the various conditions of the imaging conditions do not have to be reset one-by-one; thus, it is possible to eliminate operation hassle as a result of this.

In addition, the X-ray imaging device 1 further includes the notification unit 58 that performs notification about the deletion when the imaging conditions A, B stored in the two condition reproduction memories 53, 54 are deleted. As a result, the operator is able to know via the notification unit 58 that the imaging conditions A, B stored in the two condition reproduction memories 53, 54 have been deleted.

### [Example 2]

Example 2 of the present invention will be described next with reference to the drawings. Note that descriptions that overlap with Example 1 will be omitted. FIG. 6 illustrates a control system for lights 71, 72 and the like of an X-ray imaging device 1 according to Example 2.

In Example 1, the two memory keys 55, 56 respectively included a mechanical switch (not shown). In Example 2, the two memory keys 55, 56 include the mechanical switch (not shown), and further include two lights 71, 72 (a first light 71 and a second light 72) that illustrate the status of the respective two condition reproduction memories 53, 54. Note that the lights 71, 72 correspond to a status display unit of the present invention.

The two lights 71, 72 are respectively configured of LED, for example. An electric circuit (not shown) is interposed between the two lights 71, 72, and the lights are controlled by the primary control unit 41. The two lights 71, 72 are respectively disposed inside the mechanical switch, or disposed adjacent to the mechanical switch. The first light 71 illustrates the status of the first condition reproduction memory 53. Similarly, the second light 72 illustrates the status of the second condition reproduction memory 54.

The control of each of the lights 71, 72 by the primary control unit 41 will be described in one example of the first light 71. Note that the control of the second light 72 is the same as the control of the first light 71. The primary control unit 41 controls three operations, for example, of the first light 71. [1] When imaging conditions are not being stored in the first condition reproduction memory 53, the first light 71 is extinguished (turned OFF). [2] When the imaging conditions stored in the first condition reproduction memory 53 and the imaging conditions stored in the reference memory 51 are the same, the first light 71 turns ON. [3] When the imaging conditions stored in the first condition reproduction memory 53 and the imaging conditions stored in the reference memory 51 are different, the first light 71 flashes ON and OFF.

FIG. 7 is a flow chart for describing an operation example of the lights and the like of the X-ray imaging device according to Example 2. During Step S21, the imaging conditions A are stored in the reference memory 51, and imaging conditions are not stored in the first condition reproduction memory 53. In such a case, the light 71 is in a turned OFF state.

During Step S22, the first memory key 55 is held down, and the imaging conditions A are stored in the first condition reproduction memory 53. As a result, the imaging conditions between the imaging conditions A in the reference memory 51 and the imaging conditions A in the first condition reproduction memory 53 become the same. In such a case, the light 71 turns ON. In addition, during Step S23, the imaging conditions A in the reference memory 51 are changed to the imaging conditions B, for example, for X-ray imaging. As a result, the imaging conditions between the imaging conditions B in the reference memory 51 and the imaging conditions A in the first condition reproduction memory 53 are different. In such a case, the light 71 flashes ON and OFF.

During Step S24, the first memory key 55 is pressed normally, and the imaging conditions A in the first condition reproduction memory 53 are switched with the imaging conditions B in the reference memory 51. As a result, the imaging conditions between the imaging conditions A in the reference memory 51 and the imaging conditions A in the first condition reproduction memory 53 become the same. In such a case, the light 71 turns ON. In addition, during Step S25, when the imaging termination switch 65 on the X-ray imaging preparation screen 64 illustrated in FIG. 3 is selected, association between the current test subject and the acquired X-ray image is canceled. In conjunction with this, the reset instruction unit 57 deletes the imaging conditions A stored in the first condition reproduction memory 53. As a result, imaging conditions are no longer being stored in the first condition reproduction memory 53, and the light 71 changes to a turned OFF state.

According to the current example, the two memory keys 55, 56 include the two lights 71, 72. For example, when the imaging conditions stored in the first condition reproduction memory 53 are deleted, the first light 71 performs a display (turned OFF) different from the displays (turned ON and flashing ON and OFF) when imaging conditions are being stored in the first condition reproduction memory 53. The second light 72 also performs the same operation as the operation of the first light 71. As a result, the operator is able to know via the two lights 71,72 of the two memory keys 55, 56 that the imaging conditions in the two condition reproduction memories 53, 54 have been deleted.

Note that, for example, when the imaging conditions stored in the first condition reproduction memory 53 are deleted, the first light 71 may perform turning ON or light-flashing different from the display when imaging conditions are being stored in the first condition reproduction memory 53.

The present invention is not limited to the embodiment above, and may implement variations as described below.
(1) In each of the above-mentioned examples, the imaging termination switch 65 cancels the association between the current test subject M and the X-ray image acquired by the X-ray detector 8 and deletes the imaging conditions respectively stored in the two condition reproduction memories 53, 54. In place of this, in the present variation, the imaging initiation switch 62 performs this association and deletes the imaging conditions respectively stored in the two condition reproduction memories 53, 54.

In other words, in FIG. 4, the work of deleting the imaging conditions respectively stored in the two condition reproduction memories 53, 54, which was performed during Step S11, is performed during Step S02.

The imaging initiation switch 62 performs association between the X-ray image acquired using the X-ray detector 8 and the current test subject M. The reset instruction unit 57 erases the imaging conditions A, B stored in the two condition reproduction memories 53, 54 in conjunction with the operation that performs association via the imaging initiation switch 62. As a result, when changing between the previous test subject M to the current test subject M, the imaging conditions A, B stored in the two condition reproduction memories 53, 54 are deleted without fail.
(2) In each of the above-mentioned examples and Variation (1), two of each of the condition reproduction memories and the memory keys were respectively provided. In regards to this point, there may be one of each of the condition reproduction memories and the memory keys. In addition, there may be three or more of each of the condition reproduction memories and the memory keys.
(3) In each of the above-mentioned examples and each of the variations, the display unit 3 included two monitors 31, and was configured so as to be separate from the X-ray imaging device main body 2. Furthermore, the screen 64 and the screen 60 illustrated in FIG. 3 were displayed on one of the two monitors 31. In regards to this point, the X-ray imaging device main body 2 may include a monitor, and may display the screen 64 and the screen 60 illustrated in FIG. 3. In addition, the number of monitors 31 is not limited to two, and may be one or three or more.
(4) In each example and variation described above, the screen 60 was provided with the imaging initiation switch 62 as illustrated in FIG. 3. The screen 64 was also provided with the imaging termination switch 65. That is, the imaging initiation switch 62 and the imaging termination switch 65 were displayed using a program. In this respect, the imaging initiation switch 62 and the imaging termination switch 65 may be configured so as to include, for example, a mechanical switch referred to as a tactile switch or a push switch in, for example, the operation unit 42. Furthermore, the imaging initiation switch 62 and the imaging termination switch 65 may be operated together by one mechanical switch.
(5) In each example and variation described above, the test subject name was, for example, input by keyboard to the test subject information input field 61 of the screen 60 illustrated in FIG. 3. The screen 60 may be configured as in FIG. 8(a). That is, when the test subject information input field 61 of the screen 60 is selected, a list 81 of a plurality of test subject names appears, one test subject name (for example, a symbol DD) is selected from the list 81 of these test subject names, and when the imaging initiation switch 62 is selected after this, the device switches from the screen 60 to the screen 64.

Furthermore, the screen 60 may be configured as in FIG. 8(b). That is, the screen 60 includes a list 83 of test subject names but does not include the imaging initiation switch 62. One test subject name (for example, a symbol EE) is selected from the list 83 of test subject names displayed on the screen 60. When one test subject name is selected, the device switches from the screen 60 to the screen 64. Moreover, when one test subject name is selected, the X-ray image acquired by the X-ray detector 8 and the selected test subject name, that is, the current test subject M, are associated. That is, the imaging initiation switch 62 may be a switch that performs the association described above.
(6) In each example and variation described above, the screen 64 illustrated in FIG. 3 was provided with the imaging termination switch 65. In this respect, the imaging termination switch 65 may be a switch that cancels the association performed by the imaging initiation switch 62 or the like, and directly or indirectly returns to the screen 60. That is, the imaging termination switch 65 may be a switch that cancels the association described above.
(7) In each example and variation described above, the two memory keys 55, 56 include a mechanical switch. In this respect, the two memory keys 55, 56 may be displayed on the screen of the monitor 31 or the like. The two memory keys 55, 56 displayed on the screen are operated using, for example, a touch panel.
(8) In each example and variation described above, the X-ray imaging device main body 2 included a dolly 12. In this respect, the X-ray imaging device main body 2 may include, instead of the dolly 12, a guide member such as a rail provided on the ceiling or floor, and a moving mechanism that moves along the guide member and supports the C arm rotation and movement mechanism 11.
(9) In each example and variation described above, the two condition reproduction memories 53, 54 may be configured of one RAM or the like. Moreover, the two condition reproduction memories 53, 54 may be provided in the storage unit 43 in which the reference memory 51 is provided.

### [Description of the Reference Numerals]

- 1: X-ray imaging device
- 2: X-ray imaging device main body
- 5: X-ray tube
- 6: X-ray tube control unit
- 7: Collimator
- 8: X-ray detector
- 8B: Camera
- 8C: Detection surface
- 9: Rotation unit
- 10: C arm
- 11: Arm rotation and movement mechanism
- 31: Monitor
- 41: Primary control unit
- 42: Operation unit
- 51: Reference memory
- 53: First condition reproduction memory
- 54: Second condition reproduction memory
- 55: First memory key
- 56: Second memory key
- 57: Reset instruction unit
- 58: Notification unit
- 62: Imaging initiation switch
- 65: Imaging termination switch
- 71, 72: Light
- 83: List
- AX1: Axis

## Claims

1. An X-ray imaging device, comprising:
an X-ray source that radiates X rays toward a test subject;
a collimator that is attached to the X-ray source and that adjusts an irradiation field of the X-rays radiated from the X-ray source;
an X-ray detector that detects X-rays that have passed through the test subject;
a reference memory that stores imaging conditions including X-ray conditions and
collimator conditions, and that is referenced during X-ray imaging;
a condition reproduction memory that stores a copy of the imaging conditions stored in the reference memory;
a memory switch that causes the imaging conditions stored in the reference memory to be stored in the condition reproduction memory, and that switches the imaging conditions stored in the condition reproduction memory with the imaging conditions in the reference memory;
an operation unit that performs at least setting of the imaging conditions in the reference memory; and
a reset instruction unit that, in between a sequence of imaging a previous or next test subject and a sequence of imaging a current test subject, deletes the imaging conditions stored in the condition reproduction memory in conjunction with a preset operation of the operation unit.

2. The X-ray imaging device according to claim 1, wherein
the operation unit comprises:
an imaging initiation switch that, when initiating the sequence of imaging the current test subject, associates an X-ray image acquired via the X-ray detector and the current test subject; and
an imaging termination switch that deletes the association when terminating the sequence of imaging the current test subject, and
the reset instruction unit deletes the imaging conditions stored in the condition reproduction memory in conjunction with an operation of canceling the association performed by the imaging termination switch of the operation unit.

3. The X-ray imaging device according to claim 1, wherein
the operation unit comprises:
an imaging initiation switch that, when initiating a sequence of imaging the current test subject, associates an X-ray image acquired via the X-ray detector and the current test subject; and
an imaging termination switch that cancels the association when terminating the sequence of imaging the current test subject, and
the reset instruction unit deletes the imaging conditions stored in the condition reproduction memory in conjunction with the operation of performing association via the imaging initiation switch of the operation unit.

4. The X-ray imaging device according to claim 2 or 3, wherein
the memory switch comprises a status display unit, and
when the imaging conditions stored in the condition reproduction memory are deleted, the status display unit performs a display that is different from a display when the imaging conditions are stored in the condition reproduction memory.

5. The X-ray imaging device according to claim 2 or 3, further comprising a notification unit that, when the imaging conditions stored in the condition reproduction memory are deleted, performs notification about the deletion.

6. The X-ray imaging device according to claim 1, further comprising:
a second condition reproduction memory that stores a copy of the imaging conditions stored in the reference memory; and
a second memory switch that causes the imaging conditions stored in the reference memory to be stored in the second condition reproduction memory, and that switches the imaging conditions stored in the second condition reproduction memory with the imaging conditions in the reference memory, wherein:
the reset instruction unit deletes the imaging conditions respectively stored in the condition reproduction memory and the second condition reproduction memory.
